# EUROPÄISCHE PATENTANMELDUNG

(11) **EP 4 382 020 A1**
(43) Veröffentlichungstag der Anmeldung: **12.06.2024**
(21) Anmeldenummer: 23214408.9
(22) Anmeldetag: 05.12.2023
(51) Int. Cl.: A61B 1/00, A61B 1/04, A61B 5/01, A61B 18/00

(54) **MEDIZINISCHES INSTRUMENT MIT EINEM BILDGEBER UND VERWENDUNG**

(30) Priorität: 08.12.2022 DE 102022132715
(71) Anmelder: Karl Storz SE & Co. KG, 78532 Tuttlingen (DE)
(72) Erfinder: Brüderle, Klaus, 78532 Tuttlingen (DE)

(57) **Zusammenfassung**

Medizinisches Instrument (10) mit einem Bildgeber (14), der für elektromagnetische Strahlung zumindest mit Wellenlängen in einem ersten Wellenlängenbereich von 450nm bis 650nm und in einem zweiten Wellenlängenbereich von 9 µm bis 9,5 µm empfindlich ist, und mit einer Verarbeitungsvorrichtung (16), die dafür ausgebildet ist, eine vom Bildgeber (14) ausgegebene erste Bildinformation, die mit dem ersten Wellenlängenbereich korrespondiert, und eine zweite Bildinformation, die mit dem zweiten Wellenlängenbereich korrespondiert, derart zu verarbeiten, dass die zweite Bildinformation in erste Farbtöne umgesetzt wird und zusammen mit der ersten Bildinformation als modifizierte Bildinformation ausgegeben wird.

## Beschreibung

Die Erfindung betrifft ein medizinisches Instrument mit einem Bildgeber.

Im Bereich der Medizintechnik werden medizinische Instrumente, insbesondere Endoskope, verwendet, um innenliegende Strukturen bildhaft zu erfassen. Damit können Diagnosen erstellt oder praktische Operationen durchgeführt werden. Dies beruht hauptsächlich darauf, dass die Sachverhalte optisch, im sichtbaren Bereich, beurteilt und die Aktionen diesbezüglich ausgeführt werden.

Der Erfinder hat jedoch erkannt, dass diese optischen Erfassungen die oberflächliche Situation erfassen, also das, was mit dem menschlichen Auge zu sehen ist. Dies ist in der Regel der sichtbare Teil des Lichts.

Es ist daher eine Aufgabe der vorliegenden Erfindung ein verbessertes medizinisches Instrument aufzuzeigen, welches dem Benutzer weitergehende Informationen liefert, um Strukturen anhand einer bildhaften Darstellung erkennen und beurteilen zu können.

Die Aufgabe wird gelöst durch ein medizinisches Instrument mit einem Bildgeber, der für elektromagnetische Strahlung zumindest mit Wellenlängen in einem ersten Wellenlängenbereich von 450nm bis 650nm und in einem zweiten Wellenlängenbereich von 9 µm bis 9,5 µm empfindlich ist, und mit einer Verarbeitungsvorrichtung, die dafür ausgebildet ist, eine vom Bildgeber ausgegebene erste Bildinformation, die mit dem ersten Wellenlängenbereich korrespondiert, und eine zweite Bildinformation, die mit dem zweiten Wellenlängenbereich korrespondiert, derart zu verarbeiten, dass die zweite Bildinformation in erste Farbtöne umgesetzt wird und zusammen mit der ersten Bildinformation als modifizierte Bildinformation ausgegeben wird.

Ein solches Instrument ermöglicht es, dem Benutzer zusätzliche wichtige Information visuell zur Verfügung zu stellen. Die sichtbare Bildinformation, also die Bildinformation, die aus sichtbarem Licht gewonnen wird, wird dem Benutzer, wie bis jetzt auch, direkt zugeführt. Die thermische Bildinformation wird dem Benutzer dann per Falschfarbendarstellung als die ersten Farbtöne zugeführt. Es ist nun auch möglich, tiefe oder innere Strukturen zu erfassen, bzw. die thermische bzw. energetische Verteilung in einer Tiefenstruktur ist live darstellbar.

Dies ist insbesondere dann sehr informativ, wenn mittels entsprechender Arbeitsinstrumente Hochfrequenz (HF)-Energie zugeführt wird. Die geschieht im Rahmen einer Operation sehr oft durch HF-Geräte, die während der Operation verwendet werden. Bei der Verwendung dieser HF-Geräte erfolgt ein energetischer Eintrag bei verschieden hohen Frequenzen in das Gewebe.

Die ersten Farbtöne können grundsätzlich freigewählt werden. Es ist dabei jedoch bevorzugt diese Farbtöne in einem engen Teil des sichtbaren Spektrums zu wählen, insbesondere so eng, dass die Farbtöne in der intuitiven menschlichen Wahrnehmung als sachlich zusammengehörig verstanden werden, z.B. in einem Bereich von Hellgrün über Gelb bis Orange, von Hellblau über Mittelblau bis Dunkelblau, von Cyan über Dunkelgrün bis Hellgrün oder von Magenta über Violett bis Dunkelblau.

Die ersten Farbtöne können auch gezielt bestimmten Wellenlängen zugeordnet werden. Dabei wird ein Bildpunkt aus der zweiten Bildinformation in dem Farbton dargestellt, der laut der nachfolgenden Tabelle 1 der erfassten Wellenlänge am nächsten kommt:

| Temp [°C] | Temp [K] | λ [µm] | Farbe |
|---|---|---|---|
| 34 | 307,15 | 9,44 | dunkelblau |
| 35 | 308,15 | 9,40 | mittelblau |
| 36 | 309,15 | 9,37 | grünblau |
| 37 | 310,15 | 9,34 | grün |
| 38 | 311,15 | 9,31 | hellgrün |
| 39 | 312,15 | 9,28 | gelb |
| 40 | 313,15 | 9,25 | orange |
| 41 | 314,15 | 9,22 | rot |
| 42 | 315,15 | 9,20 | hellrot |

In Abhängigkeit von der konkreten Anwendung und der gewünschten Darstellung können diese gezielt bestimmten Wellenlängen auch über einen größeren Temperaturbereich verteilt werden. Dabei wird ein Bildpunkt aus der zweiten Bildinformation in dem Farbton dargestellt, der laut der nachfolgenden Tabelle 2 der erfassten Wellenlänge am nächsten kommt:

| Temp [°C] | Temp [K] | λ [µm] | Farbe |
|---|---|---|---|
| 40 | 313,15 | 9,25 | dunkelblau |
| 45 | 318,15 | 9,11 | mittelblau |
| 50 | 323,15 | 8,97 | grünblau |
| 55 | 328,15 | 8,83 | grün |
| 60 | 333,15 | 8,70 | hellgrün |
| 65 | 338,15 | 8,57 | gelb |
| 70 | 343,15 | 8,45 | orange |
| 75 | 348,15 | 8,32 | rot |
| 80 | 353,15 | 8,21 | hellrot |

Bei der Wahl der ersten Farbtöne wird bevorzugt berücksichtigt, dass die so erzielte Darstellung es dem Benutzer ermöglicht die Fehlfarbendarstellung der thermischen Strahlung von der Strahlung im sichtbaren Bereich des Lichts zu unterscheiden. Dabei bieten sich insbesondere Farbtöne an, die den Farben Grün und Blau zuzuordnen sind.

Es kann bevorzugt sein, dass die zweite Bildinformation unter Beibehaltung einer Intensität in die ersten Farbtöne umgesetzt wird oder dass die Intensität eines Bildpunkts der zweiten Bildinformation mit einer proportionalen Zuordnung in die Intensität des jeweiligen Farbtons umgesetzt wird.

Einige der Vorteile, die sich so realisieren lassen, werden nachfolgend beispielhaft beschrieben. Auswirkungen durch das Einbringen von HF-Energie (HF-Effekte), z.B. beim Schneiden oder Koagulieren können live sichtbar gemacht werden, insbesondere im Hinblick auf die Verteilung der HF-Energie. Da die thermische Strahlung in eine Darstellung im sichtbaren Bereich umgesetzt wird, sind Auswirkungen und Ergebnisse, die aus dem Einbringen von HF-Energie resultieren, nun als Bild dokumentierbar.

Da die thermische Strahlung in Echtzeit erfasst und auch automatisiert ausgewertet werden kann, ist es möglich eine Überwachung oder Safety-Loop über Endoskop und HF-Gerät zu realisieren. Insbesondere kann diese Falschfarbendarstellung auch per automatischer Safety-Assistenzauswertung den HF-Energieeintrag verifizieren. Zudem kann die Zusatzinformation auch als Unterstützung bei einer optischen Abgrenzung von Strukturen helfen, da der Benutzer einen größeren Spektralbereich nutzen kann und nicht nur auf eine Informationsgewinnung aus sichtbarem Licht angewiesen ist. Schließlich kann auch eine einfache Array-Temperaturmessung ermöglicht und im OP-Prozess verwendet werden.

Es wurde erläutert, dass der Bildgeber des medizinischen Instruments in einem zweiten Wellenlängenbereich von 9 µm bis 9,5 µm empfindlich ist. Bei einigen bevorzugten Ausgestaltungen ist der zweite Wellenlängenbereich von 8,5 µm bis 10,5 µm, bevorzugt von 8 µm bis 11,5 µm und besonders bevorzugt von 7,5 µm bis 13 µm. Bei einigen bevorzugten Ausgestaltungen wird als Bildgeber ein Hybridchip oder eine Hybridkamera verwendet. Diese liefern ein "sichtbares" Bild und ein "Wärmebild" mittels genau eines Bildchips. Dadurch sind die Bilder "sichtbares" Bild und "Wärmebild" zwingend kongruent, so dass Objekte/Motive automatisch positionsgleich sind.

Unter sichtbarem Licht soll hier elektromagnetische Strahlung mit einer Wellenlänge in einem Bereich zwischen 380 nm und 750 nm verstanden werden. Diese Wellenlänge typische Bereich, den das menschliche Auge erfassen kann. Das Adjektiv medizinisch im Zusammenhang mit dem medizinischen Instrument bedeutet, dass das Instrument ein Medizinprodukt ist, welches unter anderem zum Schutz von Patienten gesetzlichen Regelungen unterworfen ist, z.B. in Deutschland dem Medizinproduktegesetz oder in den USA dem Federal Food, Drug, and Cosmetic Act.

Der Begriff "empfindlich" soll hier bedeuten, dass der Bildgeber deterministisch zwischen einer ausreichenden Anzahl an verschiedenen Intensitäten der von dem beobachteten Objekt abgegebenen elektromagnetischen Strahlung im jeweiligen Wellenlängenbereich unterscheiden kann. Der Begriff "ausreichend" bedeutet dabei, dass der Benutzer die unterscheidbare Anzahl an verschiedenen Intensitäten als für seine Arbeit geeignet ansieht. Dabei sollen bevorzugt mindestens vier, mehr bevorzugt mindestens 16 und insbesondere 256 verschiedene Intensitäten unterschieden werden können. Der Bildgeber wird entsprechend dieser Vorgabe ausgewählt.

Es sei darauf hingewiesen, dass der Bildgeber auch in breiteren Wellenlängenbereichen empfindlich sein kann. Die angegebenen Wellenlängenbereiche werden jedoch als Quellen wichtiger Information angesehen, einerseits im Bereich des sichtbaren Lichts andererseits im Mittelinfrarotbereich. Daher sollte der Bildgeber zumindest in diesen Wellenlängenbereichen die vom Fachmann gewünschte Empfindlichkeit bereitstellen.

Dadurch wird die Aufgabe vollständig gelöst.

Bei einer bevorzugten Ausführungsform dämpft oder unterdrückt die Verarbeitungsvorrichtung Bildinformationen, die aufgrund von Wellenlängen gewonnen werden, die in einem Wellenlängenzwischenbereich liegen, der zwischen dem ersten Wellenlängenbereich und dem zweiten Wellenlängenbereich liegt.

Diese Ausführungsform führt dazu, dass zumindest im Wesentlichen nur elektromagnetische Strahlung aus den Wellenlängenbereichen von Interesse in der modifizierten Bildinformation enthalten ist, also dem ersten Wellenlängenbereich und dem zweiten Wellenlängenbereich. Der dazwischenliegenden Wellenlängenbereich wird hier als Ausblendbereich bezeichnet.

Geht von dem beobachteten Objekt beispielsweise weitere Infrarotstrahlung aus, die aber nicht erfasst werden soll, kann diese weitere Infrarotstrahlung unterdrückt oder zumindest gedämpft werden. Eine Infrarotstrahlung, die aber nicht erfasst werden soll, kann beispielsweise durch Fluoreszenz entstehen.

Der Ausblendbereich kann in Abhängigkeit von der gewünschten Wirkung eher eng gewählt werden, zum Beispiel von 800nm bis 850nm, 775nm bis 900 nm oder 750nm bis 950nm, oder eher breit gewählt werden, zum Beispiel 800nm bis 1000nm, 775nm bis 1500nm oder 750nm bis 2000nm. Zusätzlich kann die obere Grenze auch noch weiter erhöht werden, solange der für die gewünschte thermische Informationsgewinnung benötigte Wellenlängenbereich, insbesondere der zweite Wellenlängenbereich, nicht beeinträchtigt wird.

Bei einer weiteren bevorzugten Ausführungsform ist die Verarbeitungsvorrichtung dafür ausgebildet, eine vom Bildgeber ausgegebene dritte Bildinformation, die mit einem Wellenlängenzwischenbereich korrespondiert, der zwischen dem ersten Wellenlängenbereich und dem zweiten Wellenlängenbereich liegt, derart zu verarbeiten, dass die dritte Bildinformation zweite Farbtöne umgesetzt wird.

Diese Ausführungsform ermöglicht es, weitere Information im Infrarotbereich, insbesondere aus dem Nahinfrarotbereich, sichtbar zu machen. Die zweiten Farbtöne unterscheiden sich dabei von den ersten Farbtönen, so dass er Benutzer sofort unterscheiden kann, ob eine Farbinformation aus der modifizierten Bildinformation aus dem zweiten Wellenlängenbereich oder aus dem Wellenlängenzwischenbereich stammt.

Insbesondere können die ersten und die zweiten Farbtöne so gewählt werden, dass die ersten und die zweiten Farbtöne jeweils einer Farbe wie Rot, Grün, Blau, Braun, Violett, Gelb, Magenta, Cyan, o.ä. zugehörig sind, die ersten und die zweiten Farbtöne im Vergleich zueinander aber unterschiedlichen Farben zugehörig sind. So können beispielsweise die ersten Farbtöne der Farbe Blau zugeordnet werden und die zweiten Farbtöne der Farbe Grün zu geordnet werden.

Eine Anwendungsmöglichkeit liegt darin, dass dem Benutzer auch Infrarotstrahlung in Fehlfarben angezeigt wird, die durch Fluoreszenz hervorgerufen wird. Derartige Infrarotstrahlung kann beispielsweise für das Sichtbarmachen einer Durchblutung genutzt werden. Diese Ausführungsform ermöglicht es also, sowohl thermische Information aus dem Mittelinfrarotbereich als auch Fluoreszenzinformation aus dem Nahinfrarotbereich sichtbar zu machen. So wird dem Benutzer gleichzeitig viel Information dargestellt, so dass der Benutzer eine Situation besonders gut erkennen und bewerten kann.

Bei einer weiteren bevorzugten Ausführungsform weist der Bildgeber ein Array von für elektromagnetische Strahlung empfindlichen Elementen auf, wobei jedes für elektromagnetische Strahlung empfindliche Element einen ersten Abschnitt aufweist, der für elektromagnetische Strahlung im ersten Wellenlängenbereich empfindlich ist, und einen zweiten Abschnitt aufweist, der für elektromagnetische Strahlung im zweiten Wellenlängenbereich empfindlich ist.

Bei dieser Ausführungsform ist jedes für elektromagnetische Strahlung empfindliche Element für beide Wellenlängenbereich empfindlich. Dies ermöglicht eine kompakte Bauweise und ermöglicht ein einfaches optisches System des Instruments, da die elektromagnetische Strahlung von dem beobachteten Objekt bzw. von der Szene über genau einen optischen Pfad zum Bildgeber geführt werden kann.

Bei einer weiteren bevorzugten Ausführungsform weist der Bildgeber einen ersten Teil auf, der ein erstes Array von für elektromagnetische Strahlung empfindlichen Elementen aufweist, die für elektromagnetische Strahlung im ersten Wellenlängenbereich empfindlich sind, und weist einen zweiten Teil auf, der ein zweites Array von für elektromagnetische Strahlung empfindlichen Elementen aufweist, die für elektromagnetische Strahlung im zweiten Wellenlängenbereich empfindlich sind.

Diese Ausführungsform verwendet zwei Arrays, die jeweils elektromagnetische Information aus verschiedenen Wellenlängenbereichen, nämlich dem ersten und dem zweiten Wellenlängenbereich, erfassen. So ist es möglich, die Arrays besonders flexibel für den jeweiligen Einsatz anzupassen. Beispielsweise kann das erste Array für eine besonders hohe Auflösung ausgelegt werden, während das zweite Array eine geringere Auflösung hat, aber für den zweiten Wellenlängenbereich besonders empfindlich ist.

Die elektromagnetische Strahlung kann von dem beobachteten Objekt bzw. von der Szene über genau einen optischen Pfad zu einem Strahlteiler geführt werden, der einerseits auf das erste Array gerichtet ist und andererseits auf das zweite Array gerichtet ist.

Bei einer weiteren bevorzugten Ausführungsform ist vor dem Bildgeber ein Filter angeordnet, das dafür ausgebildet ist, Wellenlängen zwischen dem ersten und dem zweiten Wellenlängenbereich zu dämpfen.

Diese Ausführungsform führt dazu, dass der Bildgeber zumindest im Wesentlichen nur elektromagnetische Strahlung aus den Wellenlängenbereichen von Interesse erhält, also dem ersten Wellenlängenbereich und dem zweiten Wellenlängenbereich. Der dazwischenliegenden Wellenlängenbereich wird hier als Dämpfungsbereich bezeichnet.

Geht von dem beobachteten Objekt beispielsweise weitere Infrarotstrahlung aus, die aber nicht erfasst werden soll, kann diese weitere Infrarotstrahlung unterdrückt oder zumindest gedämpft werden. Eine Infrarotstrahlung, die aber nicht erfasst werden soll, kann beispielsweise durch Fluoreszenz entstehen.

Die maximale Dämpfung im Dämpfungsbereich liegt bevorzugt bei mindestens 3dB, besonders bevorzugt bei mindestens 10dB und insbesondere bei mindestens 20dB. Eine minimale Dämpfung im Dämpfungsbereich von mindestens 3dB wird bevorzugt in einem Wellenlängenbereich von 1,5 µm bis 6 µm, besonders bevorzugt 1 µm bis 7 µm und insbesondere 0,8 µm bis 8 µm erzielt.

Bei einer weiteren bevorzugten Ausführungsform kann das Instrument in einen Modus geschaltet werden, in dem die Verarbeitungsvorrichtung nur die erste Bildinformation oder nur die zweite Bildinformation als modifizierte Bildinformation ausgibt.

Diese Ausführungsform gibt dem Benutzer die Möglichkeit, nur die erste Bildinformation oder nur die zweite Bildinformation zur Darstellung auszuwählen. Dies ist insbesondere dann hilfreich, wenn eine der beiden Informationen als störend empfunden wird, oder wenn die optische Trennung zwischen der ersten Bildinformation und der zweiten Bildinformation in besonderen Situationen schwierig ist.

Bei einer weiteren bevorzugten Ausführungsform kann das Instrument in einen Modus geschaltet werden, in dem die Verarbeitungsvorrichtung wahlweise nur eine oder zwei Bildinformationen ausgewählt aus der Gruppe von erster Bildinformation, zweiter Bildinformation und dritter Bildinformation als modifizierte Bildinformation ausgibt.

Diese Ausführungsform gibt dem Benutzer die Möglichkeit, nur die erste Bildinformation, nur die zweite Bildinformation, nur die dritte Bildinformation, nur die erste und zweite Bildinformation, nur die erste und dritte Bildinformation oder nur die zweite und dritte Bildinformation zur Darstellung auszuwählen. Auch hier ist dies insbesondere dann hilfreich, wenn eine oder zwei der drei Bildinformationen als störend empfunden wird bzw. werden, oder wenn die optische Trennung zwischen den verschiedenen Bildinformationen in besonderen Situationen schwierig ist.

Bei einer weiteren bevorzugten Ausführungsform weist das Instrument ferner einen Bildschirm auf, der dafür ausgebildet ist, die modifizierte Bildinformation für einen Benutzer anzuzeigen.

Diese Ausführungsform ermöglicht es dem Benutzer die aktuelle Situation live mit den Augen zu erfassen. Der Bildschirm kann dabei insbesondere ein Monitor, ein Tablet, ein Smartphone oder Teil einer VR(virtual reality)-Brille oder AR(augmented reality)-Brille sein.

Bei einer weiteren bevorzugten Ausführungsform weist das Instrument ein Endoskop auf oder ist das Instrument als Endoskop ausgebildet.

Eine derartige Ausführungsform kann nach heutiger Einschätzung besonders von der vorliegenden Erfindung profitieren.

Ferner wird die Aufgabe gelöst durch eine Verwendung eines Bildgebers in der medizinischen Bildgebung mittels eines medizinischen Instruments, wobei der Bildgeber für elektromagnetische Strahlung zumindest mit Wellenlängen in einem ersten Wellenlängenbereich von 450nm bis 650nm und in einem zweiten Wellenlängenbereich von 9 µm bis 9,5 µm empfindlich ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiele der Erfindung ist in der Zeichnung dargestellt und wird in der nachfolgenden Beschreibung näher erläutert. Es zeigt:

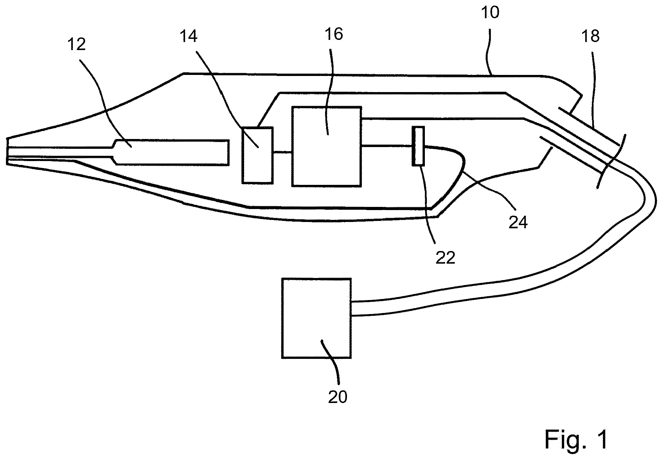

- Fig. 1: eine Ausführungsform eines medizinischen Instruments mit einem Bildgeber.

Fig. 1 zeigt ein medizinisches Instrument 10, hier ein Videoendoskop. Das medizinische Instrument 10 weist ein Linsensystem 12, das elektromagnetische Strahlung von einem zu beobachtenden Objekt bzw. von der Szene zu einem Bildgeber 14 führt. Der Bildgeber 14 ist für elektromagnetische Strahlung zumindest mit Wellenlängen in einem ersten Wellenlängenbereich von 450nm bis 650nm und in einem zweiten Wellenlängenbereich von 9 µm bis 9,5 µm empfindlich.

Das medizinische Instrument 10 weist zudem eine Verarbeitungsvorrichtung 16. Die Verarbeitungsvorrichtung 16 ist hier im Inneren des medizinischen Instruments 10 gezeigt, kann grundsätzlich aber auch außerhalb des medizinischen Instruments 10 angeordnet werden.

Die Verarbeitungsvorrichtung 16 ist dafür ausgebildet ist, eine vom Bildgeber 14 ausgegebene erste Bildinformation, die mit dem ersten Wellenlängenbereich korrespondiert, und eine zweite Bildinformation, die mit dem zweiten Wellenlängenbereich korrespondiert, derart zu verarbeiten, dass die zweite Bildinformation in erste Farbtöne umgesetzt wird und zusammen mit der ersten Bildinformation als modifizierte Bildinformation ausgegeben wird.

Bei der hier gezeigten Ausführungsform wird die modifizierte Bildinformation über einen ersten Leiter einer Kabelverbindung 18 an einen Bildschirm 20 geleitet. Grundsätzlich ist natürlich auch eine kabellose Übertragung möglich. Zudem weist die Kabelverbindung 18 hier einen optionalen zweiten Leiter auf, der direkt die Bildinformation vom Bildgeber 14 überträgt.

Es sind zudem noch eine LED 22 und ein Lichtleiter 24 dargestellt, mit denen ein zu beobachtendes Objekt bzw. eine Szene beleuchtet werden kann.

## Patentansprüche

1. Medizinisches Instrument (10) mit einem Bildgeber (14), der für elektromagnetische Strahlung zumindest mit Wellenlängen in einem ersten Wellenlängenbereich von 450nm bis 650nm und in einem zweiten Wellenlängenbereich von 9 µm bis 9,5 µm empfindlich ist, und mit einer Verarbeitungsvorrichtung (16), die dafür ausgebildet ist, eine vom Bildgeber (14) ausgegebene erste Bildinformation, die mit dem ersten Wellenlängenbereich korrespondiert, und eine zweite Bildinformation, die mit dem zweiten Wellenlängenbereich korrespondiert, derart zu verarbeiten, dass die zweite Bildinformation in erste Farbtöne umgesetzt wird und zusammen mit der ersten Bildinformation als modifizierte Bildinformation ausgegeben wird.

2. Medizinisches Instrument (10) nach Anspruch 1, wobei die Verarbeitungsvorrichtung (16) Bildinformationen dämpft oder unterdrückt, die aufgrund von Wellenlängen gewonnen werden, die in einem Wellenlängenzwischenbereich liegen, der zwischen dem ersten Wellenlängenbereich und dem zweiten Wellenlängenbereich liegt.

3. Medizinisches Instrument (10) nach Anspruch 1, wobei die Verarbeitungsvorrichtung (16), dafür ausgebildet ist, eine vom Bildgeber (14) ausgegebene dritte Bildinformation, die mit einem Wellenlängenzwischenbereich korrespondiert, der zwischen dem ersten Wellenlängenbereich und dem zweiten Wellenlängenbereich liegt, derart zu verarbeiten, dass die dritte Bildinformation in zweite Farbtöne umgesetzt wird.

4. Medizinisches Instrument (10) nach einem der vorhergehenden Ansprüche, wobei der Bildgeber (14) ein Array von für elektromagnetische Strahlung empfindlichen Elementen aufweist und wobei jedes für elektromagnetische Strahlung empfindliche Element einen ersten Abschnitt aufweist, der für elektromagnetische Strahlung im ersten Wellenlängenbereich empfindlich ist, und einen zweiten Abschnitt aufweist, der für elektromagnetische Strahlung im zweiten Wellenlängenbereich empfindlich ist.

5. Medizinisches Instrument (10) nach einem der vorhergehenden Ansprüche, wobei der Bildgeber (14) einen ersten Teil aufweist, der ein erstes Array von für elektromagnetische Strahlung empfindlichen Elementen aufweist, die für elektromagnetische Strahlung im ersten Wellenlängenbereich empfindlich sind, und einen zweiten Teil aufweist, der ein zweites Array von für elektromagnetische Strahlung empfindlichen Elementen aufweist, die für elektromagnetische Strahlung im zweiten Wellenlängenbereich empfindlich sind.

6. Medizinisches Instrument (10) nach einem der vorhergehenden Ansprüche, wobei vor dem Bildgeber (14) ein Filter angeordnet ist, das dafür ausgebildet ist, Wellenlängen zwischen dem ersten und dem zweiten Wellenlängenbereich zu dämpfen.

7. Medizinisches Instrument (10) nach einem der vorhergehenden Ansprüche, wobei das Instrument in einen Modus geschaltet werden kann, in dem die Verarbeitungsvorrichtung (16) nur die erste Bildinformation oder nur die zweite Bildinformation als modifizierte Bildinformation ausgibt.

8. Medizinisches Instrument (10) nach einem der Ansprüche 3 bis 6, wobei das Instrument in einen Modus geschaltet werden kann, in dem die Verarbeitungsvorrichtung (16) wahlweise nur eine oder zwei Bildinformationen ausgewählt aus der Gruppe von erster Bildinformation, zweiter Bildinformation und dritter Bildinformation als modifizierte Bildinformation ausgibt.

9. Medizinisches Instrument (10) nach einem der vorhergehenden Ansprüche, ferner mit einem Bildschirm (20), der dafür ausgebildet ist, die modifizierte Bildinformation für einen Benutzer anzuzeigen.

10. Medizinisches Instrument (10) nach einem der vorhergehenden Ansprüche, wobei das Instrument (10) ein Endoskop aufweist oder das Instrument als Endoskop ausgebildet ist.

11. Verwendung eines Bildgebers (14) in der medizinischen Bildgebung mittels eines medizinischen Instruments (10), wobei der Bildgeber (14) für elektromagnetische Strahlung zumindest mit Wellenlängen in einem ersten Wellenlängenbereich von 450nm bis 650nm und in einem zweiten Wellenlängenbereich von 9 µm bis 9,5 µm empfindlich ist.
